# EUROPEAN PATENT APPLICATION

(11) **EP 3 995 070 A1**
(43) Date of publication of application: **11.05.2022**
(21) Application number: 19936356.5
(22) Date of filing: 01.07.2019
(51) Int. Cl.: A61B 5/00

(54) **CENTRAL MONITORING SYSTEM, MONITORING DEVICE, MOBILE TERMINAL, AND PATIENT MONITORING METHOD**

(71) Applicant: Shenzhen Mindray Bio-Medical Electronics Co., Ltd., Shenzhen, Guangdong 518057 (CN)
(72) Inventor: LIU, Wenjun, Shenzhen, Guangdong 518057 (CN); WANG, Shengping, Shenzhen, Guangdong 518057 (CN); LIANG, Liming, Shenzhen, Guangdong 518057 (CN)
(74) Representative: KIPA AB
(86) International application number: PCT/CN2019/094251
(87) International publication number: WO 2021/000230

(57) **Abstract**

Provided are a central monitoring system (211), a monitoring device (100), a mobile terminal, and a patient monitoring method; the central monitoring system (211) obtains data from a plurality of monitoring devices (100), comprising physical sign monitoring data and patient identification data; the central monitoring system (211) displays at least part of the acquired data of the plurality of monitoring devices (100); the central monitoring system (211), in correspondence to a first event, controls at least one of the plurality of monitoring devices (100) to enter a privacy mode, while the central monitoring system (211) continues to acquire and display data from a plurality of testing devices.

## Description

### TECHNICAL FIELD

The disclosure relates to a central monitoring system, a monitoring device, a mobile terminal, and a patient monitoring method.

### BACKGROUND

Monitoring device is an instrument to monitor the vital sign data of patient, which provides a good way for the medical staff to comprehensively, intuitively and timely master the patient's condition of illness. Generally, the hospital provides a bedside monitoring device for each hospital bed. The bedside monitoring device has a screen to display data, which is convenient for the medical staff to view the monitored data. In order to facilitate the centralized management of each monitoring device by the medical staff, one or more central monitoring systems are generally configured. The central monitoring system can communicate with each monitoring device in the hospital and receive the data uploaded by the monitoring device, so that medical staff can view the monitored data of the patient's vital sign remotely at any time, which is very convenient.

The screen of the monitoring device will not only display the monitored data, such as monitored data of various vital signs, such as ECG, respiration, blood pressure, blood oxygen saturation, pulse and body temperature, as well as some alarm information, but also display some information about the identity of patient, such as ID, name, age and bed number of the patient.

Usually, just the medical staff go in and out of each inpatient ward. However, during the family visiting hours provisioned by the hospital, the patient's family members can also go in and out of the inpatient ward. In this case, the identity and vital sign data displayed by the monitoring devices of other patients will expose the personal privacy of the other patients. Therefore, most monitoring devices are equipped with the function of a privacy mode. During the family visiting hours of the hospital, the medical staff goes to each inpatient ward to manually operate the monitoring devices one after another. Such that, each monitoring device enters into the privacy mode, and the screen of the monitoring device hides and never displays some data that may involve the personal privacy. When the family visiting hours are over, the medical staff manually operate the monitoring devices one after another again to enable each monitoring device to exit the privacy mode. It can be seen that, this operation is very cumbersome, inconvenient, time-consuming and laborious. Moreover, on some occasions, such as an isolation ward, it is very inconvenient for the medical staff to enter into the isolation ward to operate the monitoring device.

### SUMMARY

The embodiments of this disclosure have provided a central monitoring system, a monitoring device, a mobile terminal, and a patient monitoring method, aiming at the above-mentioned defects.

According to a first aspect, one embodiment of this disclosure has disclosed a patient monitoring method for an in-hospital central monitoring system, which includes:
acquiring data of a plurality of monitoring devices by the central monitoring system, wherein the acquired data includes monitored data of vital sign and identity data of patient;
displaying at least part of the acquired data of the plurality of monitoring devices by the central monitoring system;
controlling at least one of the plurality of monitoring devices to enter into a privacy mode by the central monitoring system, in response to a first event;
continuing to acquire and display data of the plurality of monitoring devices by the central monitoring system.

In an embodiment, controlling at least one of the plurality of monitoring devices to enter into a privacy mode by the central monitoring system, in response to a first event, includes:
controlling at least one of the plurality of monitoring devices to enter into the privacy mode by the central monitoring system, in response to a command for entering into the privacy mode inputted by a user to the central monitoring system.

In an embodiment, controlling at least one of the plurality of monitoring devices to enter into a privacy mode by the central monitoring system, in response to a first event, includes:
controlling at least one of the plurality of monitoring devices to enter into the privacy mode by the central monitoring system, in response to reaching a preset start time for visit.

In an embodiment, the method further includes:
controlling at least one of the plurality of monitoring devices to exit the privacy mode by the central monitoring system, in response to a second event.

In an embodiment, controlling at least one of the plurality of monitoring devices to exit the privacy mode by the central monitoring system, in response to a second event, includes:
controlling at least one of the plurality of monitoring devices to exit the privacy mode by the central monitoring system, in response to a command for exiting the privacy mode inputted by a user to the central monitoring system.

In an embodiment, controlling at least one of the plurality of monitoring devices to exit the privacy mode by the central monitoring system, in response to a second event, includes:
controlling at least one of the plurality of monitoring devices to exit the privacy mode by the central monitoring system, in response to reaching a preset end time for visit.

According to a second aspect, one embodiment of this disclosure has disclosed a patient monitoring method for a medical monitoring device which is connected with a central monitoring system, wherein the method includes:
acquiring a physiological parameter acquisition signal through a sensor accessory connected with a patient, by the monitoring device;
obtaining monitored data of vital sign according to the physiological parameter acquisition signal, by the monitoring device; and
enabling the monitoring device to enter into a privacy mode in response to a first command that is sent by the central monitoring system in response to a first event, wherein the central monitoring system responds to the first event to control one or more monitoring devices to enter into the privacy mode;
continuing to use the sensor accessory connected with the patient to acquire a physiological parameter acquisition signal, obtain monitored data of vital sign according to the physiological parameter acquisition signal, and at least send the monitored data of vital sign to the central monitoring system, by the monitoring device, after entering into the privacy mode; and
hiding at least part of the monitored data of vital sign and/or identity data of patient, by the monitoring device, after entering into the privacy mode.

In an embodiment, the method further includes:
generating and displaying an area for a user to inputted a password by the monitoring device, in response to a command for entering into the privacy mode inputted by the user to the monitoring device;
determining whether the password is correct by the monitoring device, in response to the password inputted by the user in the area; and enabling the monitoring device to enter into the privacy mode when the password is correct and not to enter into the privacy mode when the password is incorrect.

In an embodiment, the method further includes:
determining whether the monitoring device is in communication connection with the central monitoring system by the monitoring device, in response to a command for entering into the privacy mode inputted by the user to the monitoring device;
enabling the monitoring device to enter into the privacy mode when the monitoring device is in communication connection with the central monitoring system and not to enter into the privacy mode when the monitoring device is not in communication connection with the central monitoring system.

In an embodiment, the method further includes:
enabling the monitoring device to exit the privacy mode when the monitoring device determines that a communication connection between the monitoring device and the central monitoring system is disconnected, after entering into the privacy mode.

In an embodiment, the monitoring device is configured with one or more display screens, and the identity data of patient includes one or more of information for bed number of patient, information for name of patient, and information for ID of patient; wherein the method further includes:
receiving, by the monitoring device, a command for patient privacy setting inputted by a user, wherein the command for patient privacy setting enables the monitoring device to select corresponding information in the identity data of patient for displaying and/or hiding;
selecting the corresponding information in the identity data of patient for the one or more display screens to display and/or hide by the monitoring device, in response to the command for patient privacy setting.

In an embodiment, the method further includes:
enabling the monitoring device to enter into a neighbor bed observation mode, in response to a command for entering into the neighbor bed observation mode inputted by a user to the monitoring device;
displaying the monitored data of vital sign and/or identity data of patient from a neighbor monitoring device corresponding to the neighbor bed observation mode by the monitoring device, when entering into the neighbor bed observation mode, wherein the identity data of patient includes at least name of patient;
hiding the name of patient in the neighbor bed observation mode by the monitoring device, in response to a command for hiding the name of patient inputted by the user.

In an embodiment, the method further includes:
enabling the monitoring device to exit the privacy mode in response to a second command that is sent by the central monitoring system in response to a second event, wherein the central monitoring system responds to the second event to control at least one of the monitoring devices to exit the privacy mode; and
displaying the hidden data again by the monitoring device after exiting the privacy mode.

In an embodiment, the method further includes:
enabling the monitoring device to exit the privacy mode, in response to reaching a preset end time for visit;
displaying the hidden data again by the monitoring device after exiting the privacy mode.

According to a third aspect, one embodiment of this disclosure has disclosed a patient monitoring method for a medical monitoring device which is in communication connection with a central monitoring system, wherein the method includes:
acquiring a physiological parameter acquisition signal through a sensor accessory connected with a patient, by the monitoring device;
obtaining monitored data of vital sign according to the physiological parameter acquisition signal, by the monitoring device; and
enabling the monitoring device to enter into a privacy mode in response to reaching a preset start time for visit;
continuing to use the sensor accessory connected with the patient to acquire the physiological parameter acquisition signal, obtain the monitored data of vital sign according to the physiological parameter acquisition signal, and at least send the monitored data of vital sign to the central monitoring system, by the monitoring device, after entering into the privacy mode; and
hiding at least part of the monitored data of vital sign and/or identity data of patient, by the monitoring device, after entering into the privacy mode.

In an embodiment, the method further includes:
generating and displaying an area for a user to inputted a password by the monitoring device, in response to a command for entering into the privacy mode inputted by the user;
determining whether the password is correct by the monitoring device, in response to the password inputted by the user in the area; and enabling the monitoring device to enter into the privacy mode when the password is correct and not to enter into the privacy mode when the password is incorrect.

In an embodiment, the method further includes:
determining whether the monitoring device is in communication connection with the central monitoring system by the monitoring device, in response to a command for entering into the privacy mode inputted by the user to the monitoring device;
enabling the monitoring device to enter into the privacy mode when the monitoring device is in communication connection with the central monitoring system and not to enter into the privacy mode when the monitoring device is not in communication connection with the central monitoring system.

In an embodiment, the method further includes:
enabling the monitoring device to exit the privacy mode when the monitoring device determines that a communication connection between the monitoring device and the central monitoring system is disconnected, after entering into the privacy mode.

In an embodiment, the monitoring device is configured with one or more display screens, and the identity data of patient includes one or more of information for bed number of patient, information for name of patient, and information for ID of patient; wherein the method further includes:
receiving a command for patient privacy setting inputted by a user to the monitoring device, by the monitoring device, wherein the command for patient privacy setting enables the monitoring device to select corresponding information in the identity data of patient for displaying and/or hiding;
selecting the corresponding information in the identity data of patient for the one or more display screens to display and/or hide by the monitoring device, in response to the command for patient privacy setting.

In an embodiment, the method further includes:
enabling the monitoring device to enter into a neighbor bed observation mode, in response to a command for entering into the neighbor bed observation mode inputted by the user to the monitoring device;
displaying the monitored data of vital sign and/or identity data of patient of a neighbor monitoring device corresponding to the neighbor bed observation mode by the monitoring device, when entering into the neighbor bed observation mode, wherein the identity data of patient includes at least name of patient;
hiding the name of patient in the neighbor bed observation mode by the monitoring device, in response to a command for hiding the name of patient inputted by the user.

In an embodiment, the method further includes:
enabling the monitoring device to exit the privacy mode in response to a second command that is sent by the central monitoring system in response to a second event, wherein the central monitoring system responds to the second event to control at least one of the monitoring devices to exit the privacy mode; and
displaying the hidden data again by the monitoring device after exiting the privacy mode.

In an embodiment, the method further includes:
enabling the monitoring device to exit the privacy mode, in response to reaching a preset end time for visit;
displaying the hidden data again by the monitoring device after exiting the privacy mode.

According to a fourth aspect, one embodiment of this disclosure has disclosed a patient monitoring method for a medical monitoring device which is in communication connection with a central monitoring system, wherein the method includes:
acquiring data of one or more neighbor bed monitoring devices by the monitoring device, wherein the data includes monitored data of vital sign and identity data of patient;
displaying at least part of the acquired data of the neighbor bed monitoring devices, by the monitoring device;
controlling at least one of the neighbor bed monitoring devices to enter into a privacy mode by the monitoring device, in response to a third event; and
continuing to acquire and display the data of the neighbor bed monitoring devices by the monitoring device.

In an embodiment, controlling at least one of the neighbor bed monitoring devices to enter into a privacy mode by the monitoring device, in response to a third event, includes:
controlling at least one of the neighbor bed monitoring devices to enter into the privacy mode by the monitoring device, in response to a command for entering into the privacy mode inputted by a user to the monitoring device.

In an embodiment, controlling at least one of the neighbor bed monitoring devices to enter into a privacy mode by the monitoring device, in response to a third event, includes:
controlling at least one of the neighbor bed monitoring devices to enter into the privacy mode by the monitoring device, in response to reaching a preset start time for visit.

In an embodiment, the method further includes:
controlling at least one of the neighbor bed monitoring devices to exit the privacy mode by the monitoring device, in response to a fourth event.

In an embodiment, controlling at least one of the neighbor bed monitoring devices to exit the privacy mode by the monitoring device, in response to a fourth event, includes:
controlling at least one of the neighbor bed monitoring devices to exit the privacy mode by the monitoring device, in response to a command for exiting the privacy mode inputted by a user to the monitoring device.

In an embodiment, controlling at least one of the neighbor bed monitoring devices to exit the privacy mode by the monitoring device, in response to a fourth event, includes:
controlling at least one of the neighbor bed monitoring devices to exit the privacy mode by the monitoring device, in response to reaching a preset end time for visit.

According to a fifth aspect, one embodiment of this disclosure has disclosed a patient monitoring method for a mobile terminal which is in communication connection with one or more monitoring devices, wherein the method includes:
controlling at least one of the monitoring devices to enter into a privacy mode by the mobile terminal, in response to a fifth event;
wherein after entering into the privacy mode, the monitoring devices continue to send data to a central monitoring system, such that the data still can be displayed in the central monitoring system.

In an embodiment, controlling at least one of the monitoring devices to enter into a privacy mode by the mobile terminal, in response to a fifth event, includes:
controlling at least one of the monitoring devices to enter into the privacy mode by the mobile terminal, in response to a command for entering into the privacy mode inputted by a user to the mobile terminal.

In an embodiment, controlling at least one of the monitoring devices to enter into a privacy mode by the mobile terminal, in response to a fifth event, includes:
controlling at least one of the monitoring devices to enter into the privacy mode by the mobile terminal, in response to reaching a preset start time for visit.

In an embodiment, the method further includes:
controlling at least one of the monitoring devices to exit the privacy mode by the mobile terminal, in response to a sixth event.

In an embodiment, controlling at least one of the monitoring devices to exit the privacy mode by the mobile terminal, in response to a sixth event, includes:
controlling at least one of the monitoring devices to exit the privacy mode by the mobile terminal, in response to a command for exiting the privacy mode inputted by a user to the mobile terminal.

In an embodiment, controlling at least one of the monitoring devices to exit the privacy mode by the mobile terminal, in response to a sixth event, includes:
controlling at least one of the monitoring devices to exit the privacy mode by the mobile terminal, in response to reaching a preset end time for visit.

According to a sixth aspect, one embodiment of this disclosure has disclosed an in-hospital central monitoring system, including:
a communication interface which is operable to establish a communication connection with a plurality of monitoring devices;
a memory which is operable to store data of the plurality of monitoring devices acquired through the communication interface and to store a program, wherein the data includes monitored data of vital sign and identity data of patient;
a display which is operable to display the acquired data of the monitoring devices; and
a processor which is operable to execute the program stored in the memory to implement the patient monitoring method for an in-hospital central monitoring system, recited in any one embodiment discussed above.

According to a seventh aspect, one embodiment of this disclosure has disclosed a medical monitoring device, which includes:
a signal acquisition circuit which is operable to use a sensor accessory connected with a patient to acquire a physiological parameter acquisition signal;
a processor which is operable to obtain monitored data of vital sign according to the physiological parameter acquisition signal acquired by the signal acquisition circuit;
a memory which is operable to store the monitored data of vital sign and identity data of patient; and
a display which is operable to display data;
wherein the memory is further operable to store a program, and the processor is further operable to execute the program stored in the memory to implement patient monitoring method for a medical monitoring device, recited in any one embodiment discussed above, and some improvement functions of the monitoring device itself related to a privacy mode, and so on.

According to an eighth aspect, one embodiment of this disclosure has disclosed a mobile terminal, which includes:
a communication interface which is operable to communicationally connect with a plurality of monitoring devices;
a memory which is operable to store a program; and
a processor which is operable to execute the program stored in the memory to implement the patient monitoring method for a mobile terminal, recited in any one embodiment discussed above.

According to a ninth aspect, one embodiment of this disclosure has disclosed a computer storage medium, which includes a program that can be executed by a processor to implement any one method described above.

### BRIEF DESCRIPTION OF THE DRAWINGS

Fig. 1 is a structural diagram of a monitoring device according to an embodiment.
Fig. 2 is a structural diagram of a monitoring device according to another embodiment.
Fig. 3 is a structural diagram of a monitoring system according to an embodiment.
Fig. 4 is a flowchart of a patient monitoring method for an in-hospital central monitoring system according to an embodiment.
Fig. 5 is a flowchart of a patient monitoring method for an in-hospital central monitoring system according to another embodiment.
Fig. 6 is a structural diagram of an in-hospital central monitoring system according to an embodiment.
Fig. 7 is a flowchart of a patient monitoring method for a medical monitoring device according to an embodiment.
Fig. 8 is a flowchart of a patient monitoring method for a medical monitoring device according to another embodiment.
Fig. 9 is a flowchart of a patient monitoring method for a medical monitoring device according to a further embodiment.
Fig. 10 is a flowchart of a patient monitoring method for a mobile terminal according to an embodiment.
Fig. 11 is a structural diagram of a mobile terminal according to an embodiment.
Fig. 12 is a flowchart of a patient monitoring method for a medical monitoring device according to another embodiment.
Fig. 13 is a flowchart of a patient monitoring method for a medical monitoring device according to a further embodiment.

### DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the specification are operable to distinguish different objects, rather than to describe a specific order. In addition, the terms "connection", and "linkage" mentioned in this application include direct and indirect connection (linkage), unless otherwise specified.

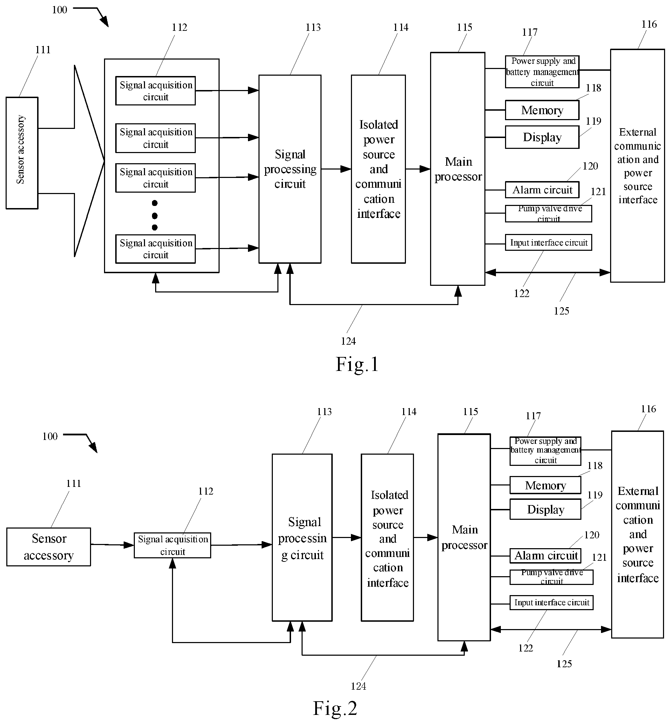

Fig. 1 is a system framework diagram of a monitoring device 100 for multi-parameter monitoring. The monitoring device 100 can include an independent housing. A housing panel has a sensor interface area in which multiple sensor interfaces are integrated for connecting with external physiological parameter sensor accessories 111. The housing panel further includes a small IXD display area, a display 119, an input interface circuit 122, and an alarm circuit 120 (such as an LED alarm area), etc. The monitoring device 100 can have an external communication and power source interface 116 for communicating with a main unit and taking power from the main unit. The monitoring device 100 also supports a build-out parameter module, can form a plug-in monitor main unit by means of inserting the parameter module, can be used as part of the monitoring device, or can be connected to the main unit via a cable, with the build-out parameter module being used as an external accessory of the monitoring device.

The internal circuit of the monitoring device 100 is disposed in the housing. As shown in Fig. 1 the internal circuit includes signal acquisition circuits 112 corresponding to at least two physiological parameters, a front-end signal processing circuit 113, and a main processor 115. The signal acquisition circuits 112 may be selected from an electrocardiogram circuit, a respiration circuit, a body temperature circuit, a blood oxygen saturation circuit, a non-invasive blood pressure circuit, an invasive blood pressure circuit, etc. The signal acquisition circuits 112 are respectively electrically connected to the corresponding sensor interfaces and are used to be electrically connected to the sensor accessories 111 corresponding to different physiological parameters, with an output end thereof being coupled to the front-end signal processing circuit 113. A communication port of the front-end signal processing circuit 113 is coupled to the main processor 115, and the main processor 115 is electrically connected to the external communication and power source interface 116. Various physiological parameter measurement circuits can use common circuits in the prior art, the front-end signal processing circuit 113 completes the sampling and analog-to-digital conversion of the output signal of the signal acquisition circuit 112, and outputs control signals to control the measurement process of the physiological signals. These parameters include but are not limited to parameters of electrocardiogram, respiration, body temperature, blood oxygen saturation, non-invasive blood pressure, and invasive blood pressure. The front-end signal processing circuit 113 can be realized by a single-chip microcomputer (such as mixed signal single chip microcomputer of LPC2136 from PHLIPS company or ADuC7021 from ADI, and so on) or other semiconductor devices and can also be realized by an ASIC or FPGA. The front-end signal processing circuit 113 can be powered by an isolated power source. The sampled data is simply processed and packaged, and then sent to the main processor through the isolated communication interface. For example, the front-end signal processing circuit 113 can be coupled to the main processor 115 through the isolated power source and communication interface 114. The reason that the front-end signal processing circuit 113 is powered by an isolated power source is that the DC/DC power source isolated by a transformer plays a role in isolating the patient from the power supply device, and the main purpose is: 1. isolating the patient, in which the application part is floated above the ground through the isolation transformer such that the leakage current of the patient is small enough; and 2. preventing the voltage or energy in the application of defibrillation or electric scalpel from affecting the boards and devices of the intermediate circuit such as the main control board (guaranteed by creepage distance and electrical clearance). Of course, the front-end signal processing circuit 113 may also be connected to the main processor 115 through a cable 124. The main processor 15 completes the calculation of physiological parameters and sends the calculation results and waveforms of the parameters to the main unit (such as a main unit with a display, PC, central station, etc.) through the external communication and power source interface 16. The main processor 15 is connected with the external communication and power source interface 116 through a cable 125 for communicating and taking power. A power supply and battery management circuit 117, which takes power from the main unit through the external communication and power interface 116 and supplies the power to the main processor 115 after processing, such as rectification and filtering, is provided. The power supply and battery management circuit 117 can also monitor, manage and protect the power obtained from the main unit through the external communication and power interface 116. The external communication and power source interface 116 may be one or a combination of local area network interfaces composed of Ethernet, Token Ring, Token Bus, and the optical fibre distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication and power source interface 116 may also be one or a combination of the wireless data transmission interface and the wired data transmission interface. The main unit can be any computer device, such as the main unit of the monitoring device 100, ECG machine, ultrasonic diagnostic instrument and computer. The monitoring device 100 can be formed by installing the matching software on the main unit. The main unit can also be a communication device, such as a mobile phone. The parameter processing module sends the data to the mobile phone supporting Bluetooth communication through the Bluetooth interface to realize the remote transmission of data. After the main processor 115 completes the calculation of physiological parameters, it can also determine whether the physiological parameters are abnormal. If yes, it can alarm through the alarm circuit 120. The memory 118 may store the intermediate and final data of the monitoring device 100 and program instructions or code for execution by the main processor 115 or the like. If the monitoring device 100 has the function of blood pressure measurement, it may also include a pump valve drive circuit 121 for inflation or deflation under the control of the main processor 115.

The monitoring device 100 shown in Fig. 1 is a multi-parameter monitoring device. Of course, the monitoring device 100 can also be a monitoring device for a single physiological parameter. Fig. 2 is an example for such monitoring device, and the same content can be seen in Fig. 1 above.

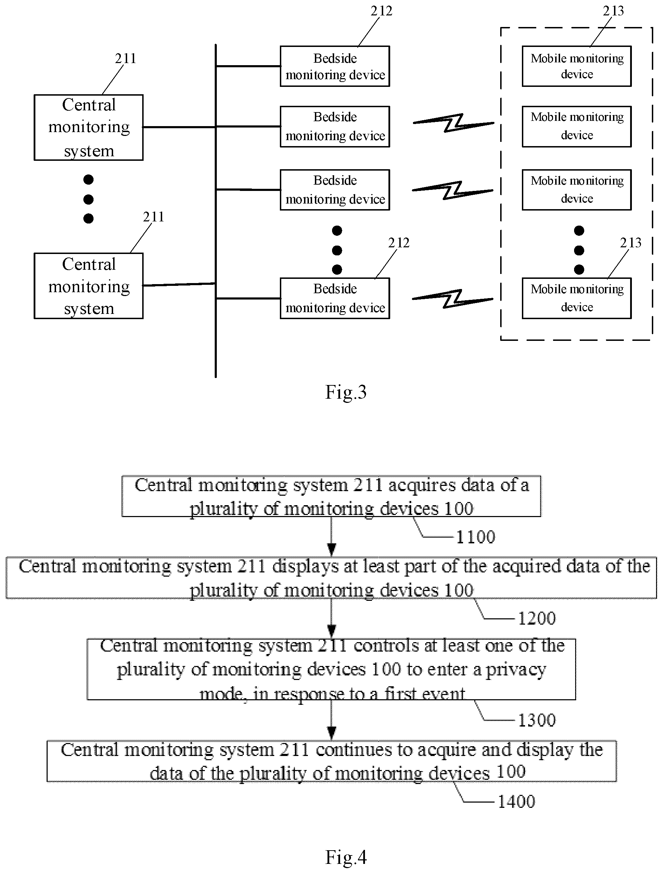

As shown in Fig. 3, a monitoring system is provided. By using the monitoring device system, the data of a monitoring device may be saved as a whole, where patient information and nursing information can be managed centrally and may be stored in association, which facilitates the storage of historical data and associated alarming. The monitoring device 100 can be divided into two types, one of which is a bedside monitoring device 212 and the other one of which is a mobile monitoring device 213. In the system shown in Fig. 3, a bedside monitoring device 212 can be provided for each hospital bed and can be the above-mentioned multi-parameter monitoring device or plug-in monitoring device. In some examples, the bedside monitoring device 212 can also be paired with the mobile monitoring device 213. The mobile monitoring device 213 provides a simple and portable monitoring function, as it can be worn on the body of a patient for mobile monitoring of the patient. With the wired or wireless communication between the mobile monitoring device 213 and the bedside monitoring device 212, the monitored data of vital sign generated during the mobile monitoring can be transmitted to the bedside monitoring device 212 for displaying or transmitted to the central monitoring system 211 through the bedside monitoring device 212 for doctors or nurses to view. In addition, the mobile monitoring device 213 may further directly transmit, through a wireless network arranged in the hospital, the monitored data of vital sign generated during the mobile monitoring process to the central monitoring system 211 for storage and display. It can be seen that, the data which is corresponding to the monitored data of vital sign and is displayed on the bedside monitoring device 212 may originate from the sensor accessory directly connected to the monitoring device, or from the mobile monitoring device 213. Similarly, the data which is corresponding to the monitored data of vital sign and is viewed by the doctors or nurses on the central monitoring system 211 may also originate from the bedside monitoring device 212, or from the mobile monitoring device 213.

The above is a description of the central monitoring system 211 and the monitoring device 100. Considering the inconvenience operation of the medical staff for enabling the monitoring device 100 to enter into and exit the privacy mode, especially in isolated wards, a scheme that can remotely control the monitoring device 100 to enter into and exit the privacy mode is proposed in this disclosure. Further, considering the cumbersome, time-consuming and laborious operation for enabling the monitoring device 100 to enter into and exit the privacy mode, in some schemes, a scheme that can remotely control a batch of monitoring devices 100 to enter into and exit the privacy mode is provided. The details are as follows.

The scheme about controlling the monitoring device 100 to enter into and exit the privacy mode by the central monitoring system is described below.

Referring Fig.4, one embodiment of this disclosure has disclosed a patient monitoring method for an in-hospital central monitoring system, which includes steps 1100 and 1400.

In step 1100, the central monitoring system 211 acquires data of a plurality of monitoring devices 100, wherein the data includes monitored data of vital sign and identity data of patient. The monitored data of vital sign in this disclosure refers to some data used to represent the user's physical signs, which can include physiological parameter values and physiological waveform data, as well as the data obtained by further processing the physiological parameter values and physiological waveform data, such as some alarm data, etc. The identity data of patient refers to some data used to represent the identity of patient, such as information about the identity of patient, such as ID, name, age and bed number of the patient.

In step 1200, the central monitoring system 211 displays at least part of the acquired data of the plurality of monitoring devices 100.

In step 1300, the central monitoring system 211 controls at least one of the plurality of monitoring devices 100 to enter into a privacy mode, in response to a first event. In some examples, the first event may be a command for entering into the privacy mode inputted by a user to the central monitoring system. In other examples, the first event may be reaching a preset start time for visit. These embodiments will be described in detail below.

In one embodiment, the central monitoring system 211 controls at least one of the plurality of monitoring devices 100 to enter into a privacy mode, in response to a first event, includes that the central monitoring system 211 controls at least one of the plurality of monitoring devices 100 (such as one or more monitoring devices 100) to enter into a privacy mode, in response to a command for entering into the privacy mode inputted by a user to the central monitoring system 211. In one embodiment, the command for entering into the privacy mode is generated by triggering a physical or virtual key of the central monitoring system 211. Or the command for entering into the privacy mode is generated by recognizing a gesture or voice of the user by the central monitoring system 211. For example, the central monitoring system provides a physical or virtual key. When the user presses the key, the central monitoring system 211 controls at least one of the plurality of monitoring devices 100 to enter into the privacy mode. For another example, the user sends a voice to the central monitoring system 211, such as "controlling the monitoring device to enter into the privacy mode". After recognizing the voice, the central monitoring system 211 controls at least one of the plurality of monitoring devices 100 to enter into the privacy mode. For another example, the user sends a preset action, such as waving a hand, to the central monitoring system 211. After recognizing the preset action, the central monitoring system 211 controls at least one of the plurality of monitoring devices 100 to enter into the privacy mode.

In one embodiment, the central monitoring system 211 controls at least one of the plurality of monitoring devices 100 to enter into a privacy mode, in response to a first event, includes that the central monitoring system 211 controls at least one of the plurality of monitoring devices 100 to enter into a privacy mode, in response to reaching a preset start time for visit. For example, the preset visit start time is 5 p.m. When it is 5 p.m., the central monitoring system 211 automatically controls at least one of the plurality of monitoring devices 100 to enter into the privacy mode.

In step 1400, the central monitoring system 211 continues to acquire and display the data of the plurality of monitoring devices 100. After the monitoring device 100 enters into the privacy mode, the monitoring device 100 will hide and not display many data that may leak the patient's privacy. Accordingly, for the sake of monitoring safety, the central monitoring system 211 should continue to obtain and display the data of the monitoring device when the medical staff want to view, especially for these monitoring devices entering into the privacy mode. At this time, of course, the data of the monitoring device obtained by the central monitoring system 211 can also include the data that is hidden and not displayed in the monitoring device itself. Which data should be hidden and which data can be displayed can be preset by the manufacturer at the factory in some cases, and can also be preset by the medical staff in some cases.

In some examples, the central monitoring system 211 can be configured with one or more display screens. The central monitoring system 211 receives the command for entering into the privacy mode inputted by the user to the central monitoring system 211. The command for entering into the privacy mode is used to enable the central monitoring system 211 to select the corresponding information in the identity data of patient to display and/or hide. Therefore, in response to the command for entering into the privacy mode, the central monitoring system 211 selects the corresponding information in the identity data of patient for one or more display screens to display and/or hide. As described above, the identity data of patient is some data used to represent the patient's own identity, such as one or more items of the information about the identity of patient, such as ID, name, age and bed number of the patient. Therefore, the medical staff can select which information in the identity data of patient to be displayed for each display screen of the central monitoring system 211. For example, the medical staff can set the display screens configured in public areas such as aisles and corridors to display only the patient ID in the identity data of patient, or to hide and do not display the patient ID and patient name, while set the display screens configured in more private areas such as department offices to display all the information in the identity data of patient and do not hide any information in the identity data of patient.

When the information about the name of patient is selected to be hidden, the central monitoring system 211 can also provide a physical or virtual key. When the central monitoring system 211 determines that the key is in a pressed state, the central monitoring system 211 displays the information about the name of patient. When the central monitoring system 211 determines that the key exits the pressed state, the central monitoring system 211 continues to hide the information about the name of patient. In a typical scene, when the user wants to view the hidden name of patient on the display screen of the central monitoring system 211 in the aisle, he/she presses down the above key and keeps pressing, then the display screen of the aisle will display the hidden name of patient. When the user releases his/her hand, the key will bounce up again, the display screen in the aisle will hide the name of patient again. In such a way, the function of temporarily displaying the name of patient is realized. Of course, in another interactive logic, when the central monitoring system 211 hides the information about the name of patient, the central monitoring system 211 displays the information about the name of patient when it receives the signal that the key is triggered. When the central monitoring system 211 displays the information about the name of patient, the central monitoring system 211 hides the information about the name of patient when it receives the signal that the key is triggered. In a typical scene, in the situation that the display screen of the central monitoring system 211 in the aisle hides the information about the name of patient, the display screen in the aisle will display the information about the name of patient, if the user presses the key. Then the display screen in the aisle will hides the information about the name of patient, if the user presses the key again. In such a way, the function of temporarily displaying the name of patient is also realized. In some examples, the central monitoring system 211 can also enable the key in response to an enabling command, that is, the key is valid, and the user can temporarily view the information about the name of patient through the key. And the central monitoring system 211 can also disable the key in response to a disabling command, that is, the key is invalid, and the user cannot temporarily view the information about the name of patient through the key.

The above are some examples where the central monitoring system 211 remotely controls a single or a batch of monitoring devices 100 to enter into the privacy mode. The following describes the case where the central monitoring system 211 remotely controls a single or a batch of monitoring devices 100 to exit the privacy mode.

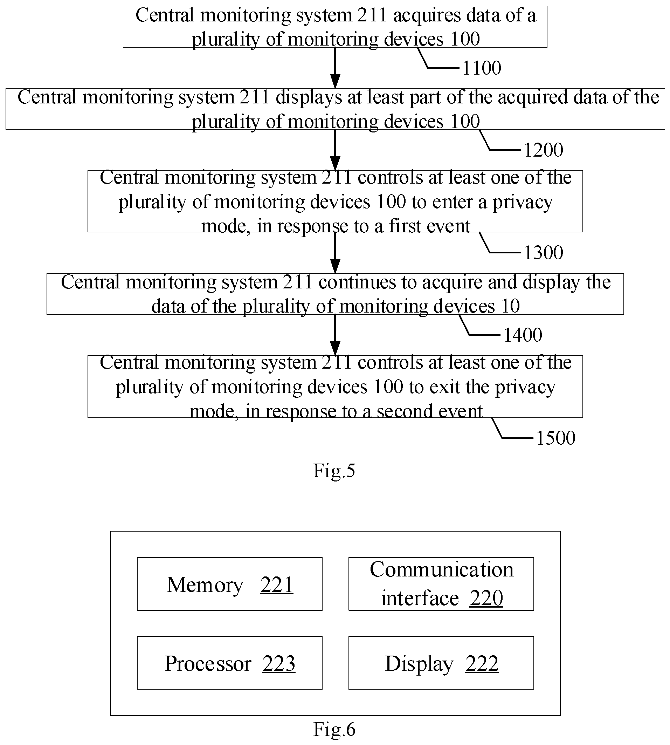

Referring Fig.5, one embodiment of this disclosure has disclosed a patient monitoring method for an in-hospital central monitoring system, which includes step 1500, in additional to steps 1100 and 1400.

In step 1500, the central monitoring system 211 controls at least one of the plurality of monitoring devices 100 to exit the privacy mode, in response to a second event. In some examples, the second event can be a command for exiting the privacy mode inputted by a user to the central monitoring system 211. In some other examples, the second event can be reaching a preset end time for visit. These embodiments will be described in detail below.

In an embodiment, the central monitoring system 211 controls at least one of the plurality of monitoring devices 100 to exit the privacy mode, in response to a second event, includes the central monitoring system 211 controls at least one of the plurality of monitoring devices 100 (such as one or more monitoring devices 100) to exit the privacy mode, in response to a command for exiting the privacy mode inputted by a user to the central monitoring system 211. In one embodiment, the command for exiting the privacy mode is generated by triggering a physical or virtual key of the central monitoring system 211. Or the command for exiting the privacy mode is generated by recognizing the gesture or voice of the user by the central monitoring system 211. For example, the central monitoring system provides a physical or virtual key. When the user presses the key, the central monitoring system 211 controls at least one of the plurality of monitoring devices 100 to exit the privacy mode. For another example, the user sends a voice to the central monitoring system 211, such as "controlling the monitoring device to exit the privacy mode". After recognizing the voice, the central monitoring system 211 controls at least one of the plurality of monitoring devices 100 to exit the privacy mode. For another example, the user sends a preset action, such as shaking his/her head, to the central monitoring system 211. After recognizing the preset action, the central monitoring system 211 controls at least one of the plurality of monitoring devices 100 to exit the privacy mode.

In one embodiment, the central monitoring system 211 controls at least one of the plurality of monitoring devices 100 to exit a privacy mode, in response to a second event, includes that the central monitoring system 211 controls at least one of the plurality of monitoring devices 100 to exit a privacy mode, in response to reaching a preset end time for visit. For example, the preset visit end time is 7 p.m. When it is 7 p.m., the central monitoring system 211 automatically controls at least one of the plurality of monitoring devices 100 to exit the privacy mode.

Referring Fig.6, one embodiment of this disclosure has disclosed an in-hospital central monitoring system 211, which includes a communication interface 220, a memory 221, a display 222 and a processor 223. Wherein the communication interface 220 is operable to establish a communication connection with a plurality of monitoring devices 100. The memory 221 is operable to store data of the plurality of monitoring devices 100 acquired through the communication interface 220 and to store a program, wherein the data includes monitored data of vital sign and identity data of patient. The display 222 is operable to display the acquired data of the monitoring devices 100. The processor 223 is operable to execute the program stored in the memory 221 to implement the patient monitoring method for an in-hospital central monitoring system 211, recited in any one embodiment discussed herein.

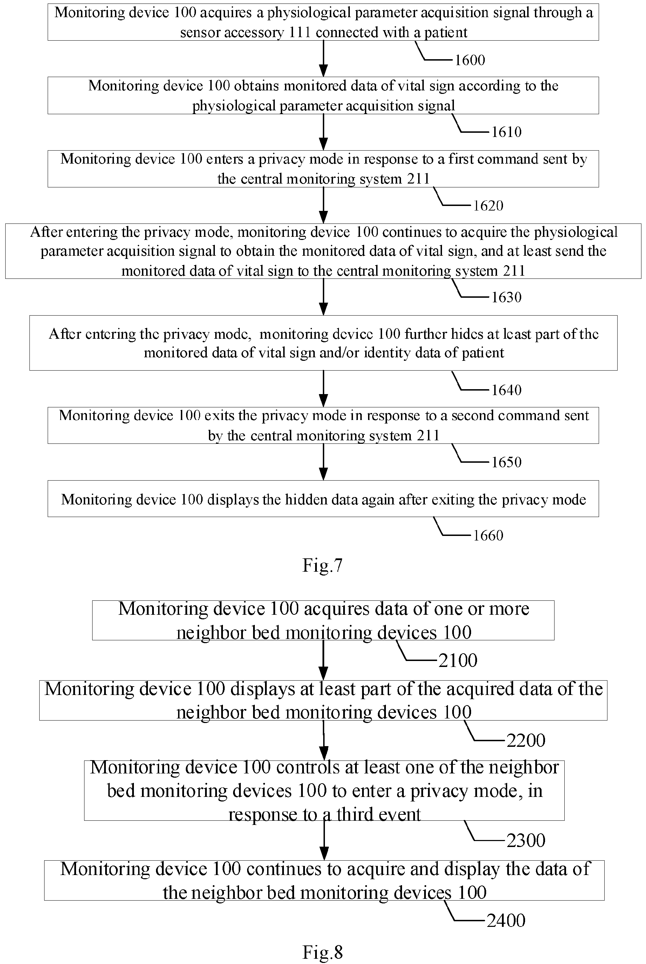

Correspondingly, referring Fig.7, one embodiment of this disclosure has disclosed a patient monitoring method for a medical monitoring device which is connected with a central monitoring system. The patient monitoring method can include steps 1600 and 1640. In some embodiments, the patient monitoring method can further include steps 1650 and 1660.

In step 1600, the monitoring device 100 acquires a physiological parameter acquisition signal through a sensor accessory 111 connected with a patient.

In step 1610, the monitoring device 100 obtains monitored data of vital sign according to the physiological parameter acquisition signal.

In step 1620, the monitoring device 100 enters into a privacy mode in response to a first command sent by the central monitoring system 211, wherein the first command is sent by the central monitoring system 211 in response to a first event. According to the description above, it is known that the first event is responded by the central monitoring system 211 to control one or more monitoring devices 100 to enter into the privacy mode. Accordingly, the first event is not further described herein for concise.

In step 1630, after entering into the privacy mode, the monitoring device 100 continues to use the sensor accessory 111 connected with the patient to acquire the physiological parameter acquisition signal, obtain the monitored data of vital sign according to the physiological parameter acquisition signal, and at least send the monitored data of vital sign to the central monitoring system 211.

In step 1640, after entering into the privacy mode, the monitoring device 100 further hides at least part of the monitored data of vital sign and/or identity data of patient. For example, the monitoring device 100 can hide the monitored data of vital sign and display the identity data of patient.

In step 1650, the monitoring device 100 can exit the privacy mode in response to a second command sent by the central monitoring system 211, wherein the second command is sent by the central monitoring system 211 in response to a second event, and the second event is responded by the central monitoring system 211. According to the description above, it is known that the second event is responded by the central monitoring system 211 to control at least one of the plurality monitoring devices 100 to exit the privacy mode. Accordingly, the second event is not further described herein for concise.

In step 1660, the monitoring device 100 displays the hidden data again after exiting the privacy mode.

The scheme that the monitoring device 100 controls neighbor monitoring device 100 to enter into and exit the privacy mode is described below.

Referring Fig.8, one embodiment of this disclosure has disclosed a patient monitoring method for a medical monitoring device, which is in communication connection with a central monitoring system 211. The patient monitoring method for a medical monitoring device includes steps 2100 and 2400.

In step 2100, the monitoring device 100 acquires data of one or more neighbor bed monitoring devices 100, wherein the data includes monitored data of vital sign and identity data of patient.

In step 2200, the monitoring device 100 displays at least part of the acquired data of the neighbor bed monitoring devices 100.

In step 2300, the monitoring device 100 controls at least one of the neighbor bed monitoring devices 100 to enter into a privacy mode, in response to a third event. In some examples, the third event may be a command for entering into the privacy mode inputted by a user to the monitoring device 100. In other examples, the third event may be reaching a preset start time for visit. These embodiments will be described in detail below.

In an embodiment, the monitoring device 100 controls at least one of the neighbor bed monitoring devices 100 to enter into a privacy mode, in response to a third event, includes: the monitoring device 100 controls at least one of the neighbor bed monitoring devices 100 (such as one or more neighbor bed monitoring devices 100) to enter into the privacy mode, in response to a command for entering into the privacy mode inputted by a user to the monitoring device 100. In one embodiment, the command for entering into the privacy mode is generated by triggering a physical or virtual key of the monitoring device 100. Or the command for entering into the privacy mode is generated by recognizing the gesture or voice of the user by the monitoring device 100. For example, the monitoring device 100 provides a physical or virtual key. When the user presses the key, the monitoring device 100 controls at least one of the plurality of neighbor bed monitoring devices 100 to enter into the privacy mode. For another example, the user sends a voice to the monitoring device 100, such as "controlling the monitoring device to enter into the privacy mode". After recognizing the voice, the monitoring device 100 controls at least one of the plurality of neighbor bed monitoring devices 100 to enter into the privacy mode. For another example, the user sends a preset action, such as waving a hand, to the monitoring device 100. After recognizing the preset action, the monitoring device 100 controls at least one of the plurality of neighbor bed monitoring devices 100 to enter into the privacy mode.

In one embodiment, the monitoring device 100 controls at least one of the plurality of neighbor bed monitoring devices 100 to enter into a privacy mode, in response to a first event, includes that the monitoring device 100 controls at least one of the plurality of neighbor bed monitoring devices 100 to enter into a privacy mode, in response to reaching a preset start time for visit. For example, the preset visit start time is 5 p.m. When it is 5 p.m., the monitoring device 100 automatically controls at least one of the plurality of neighbor bed monitoring devices 100 to enter into the privacy mode.

In step 2400, the monitoring device 100 continues to acquire and display the data of the neighbor bed monitoring devices 100.

The above are some examples where the monitoring device 100 remotely controls a single or a batch of neighbor bed monitoring devices 100 to enter into the privacy mode. The following describes the case where the monitoring device 100 remotely controls a single or a batch of neighbor bed monitoring devices to exit the privacy mode.

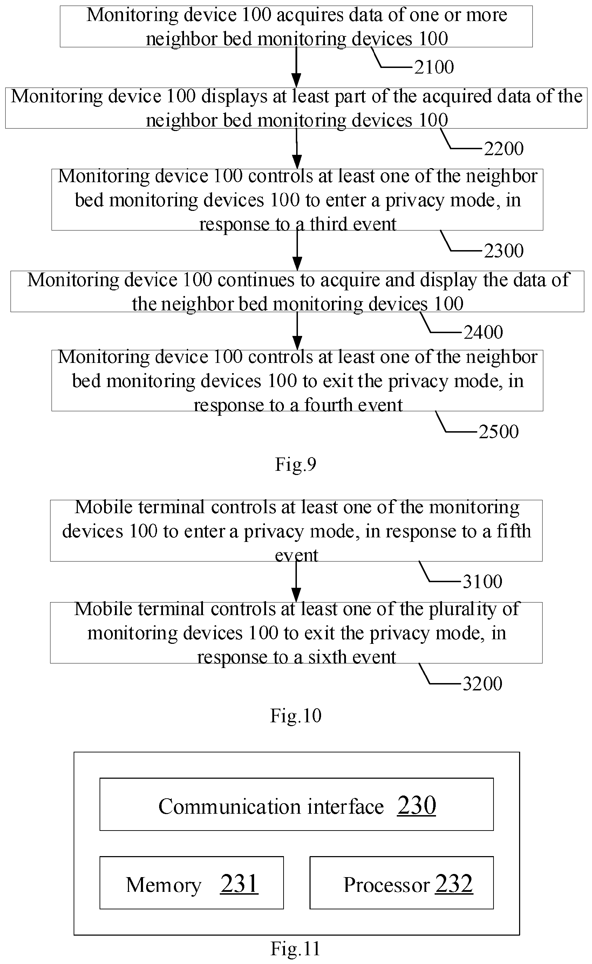

Referring Fig.9, one embodiment of this disclosure has disclosed a patient monitoring method for a medical monitoring device, which includes step 2500, in additional to steps 2100 and 2400.

In step 2500, the monitoring device 100 controls at least one of the neighbor bed monitoring devices 100 to exit the privacy mode, in response to a fourth event. In some examples, the fourth event may be a command for exiting the privacy mode inputted by a user to the monitoring device 100. In other examples, the fourth event may be reaching a preset end time for visit. These embodiments will be described in detail below.

In an embodiment, the monitoring device 100 controls at least one of the neighbor bed monitoring devices 100 to exit the privacy mode, in response to a fourth event, includes, the monitoring device 100 controls at least one of the neighbor bed monitoring devices 100 (such as one or more neighbor bed monitoring devices 100) to exit the privacy mode, in response to a command for exiting the privacy mode inputted by a user to the monitoring device 100. In one embodiment, the command for exiting the privacy mode is generated by triggering a physical or virtual key of the monitoring device 100. Or the command for exiting the privacy mode is generated by recognizing the gesture or voice of the user by the monitoring device 100. For example, the monitoring device 100 provides a physical or virtual key. When the user presses the key, the monitoring device 100 controls at least one of the plurality of neighbor bed monitoring devices 100 to exit the privacy mode. For another example, the user sends a voice to the monitoring device 100, such as "controlling the monitoring device to exit the privacy mode". After recognizing the voice, the monitoring device 100 controls at least one of the plurality of neighbor bed monitoring devices 100 to exit the privacy mode. For another example, the user sends a preset action, such as shaking his/her head, to the monitoring device 100. After recognizing the preset action, the monitoring device 100 controls at least one of the plurality of neighbor bed monitoring devices 100 to exit the privacy mode.

In an embodiment, the monitoring device 100 controls at least one of the neighbor bed monitoring devices 100 to exit the privacy mode, in response to a fourth event, includes, the monitoring device 100 controls at least one of the neighbor bed monitoring devices 100 to exit the privacy mode, in response to preset visit end time which is up. For example, the preset visit end time is 7 p.m. When it is 7 p.m., the monitoring device 100 automatically controls at least one of the plurality of neighbor bed monitoring devices to exit the privacy mode.

The scheme that the mobile terminal, such as a mobile phone or a tablet computer and so on, controls the monitoring device 100 to enter into and exit the privacy mode is described below.

Referring Fig. 10, one embodiment of this disclosure has disclosed a patient monitoring method for a mobile terminal, which is in communication connection with one or more monitoring devices 100. The patient monitoring method for a mobile terminal includes steps 3100 and/or 3200.

In step 3100, the mobile terminal controls at least one of the monitoring devices 100 to enter into a privacy mode, in response to a fifth event. After entering into the privacy mode, the monitoring device 100 continues to send data to the central monitoring system 211, such that the data still can be displayed in the central monitoring system 211. In some examples, the fifth event may be a command for entering into the privacy mode inputted by a user to the mobile terminal. In other examples, the fifth event may be reaching a preset start time for visit. These embodiments will be described in detail below.

In one embodiment, the mobile terminal controls at least one of the plurality of monitoring devices 100 to enter into a privacy mode, in response to a fifth event, includes that the mobile terminal controls at least one of the plurality of monitoring devices 100 to enter into a privacy mode, in response to a command for entering into the privacy mode inputted by a user to the mobile terminal. In one embodiment, the command for entering into the privacy mode is generated by triggering a physical or virtual key of the mobile terminal. Or the command for entering into the privacy mode is generated by recognizing the voice of the user by the mobile terminal. Or the command for entering into the privacy mode is generated by shaking the mobile terminal. Or the command for entering into the privacy mode is generated by recognizing the touching gesture of the user on the mobile terminal, by the mobile terminal. For example, the mobile terminal provides a physical or virtual key. When the user presses the key, the mobile terminal controls at least one of the plurality of monitoring devices 100 to enter into the privacy mode. For another example, the user sends a voice to the mobile terminal, such as "controlling the monitoring device to enter into the privacy mode". After recognizing the voice, the mobile terminal controls at least one of the plurality of monitoring devices 100 to enter into the privacy mode. For another example, after the user shakes the mobile terminal, the mobile terminal controls at least one of the plurality of monitoring devices 100 to enter into the privacy mode. For a further example, after recognizing the preset touching gesture of the user, the mobile terminal controls at least one of the plurality of monitoring devices 100 to enter into the privacy mode.

In one embodiment, the mobile terminal controls at least one of the plurality of monitoring devices 100 to enter into a privacy mode, in response to a fifth event, includes that the mobile terminal controls at least one of the plurality of monitoring devices 100 to enter into a privacy mode, in response to reaching a preset start time for visit. For example, the preset visit start time is 5 p.m. When it is 5 p.m., the mobile terminal automatically controls at least one of the plurality of monitoring devices 100 to enter into the privacy mode.

The above are some examples where the mobile terminal remotely controls a single or a batch of monitoring devices 100 to enter into the privacy mode. The following describes the case where the mobile terminal remotely controls a single or a batch of monitoring devices 100 to exit the privacy mode.

In step 3200, the mobile terminal controls at least one of the plurality of monitoring devices 100 to exit the privacy mode, in response to a sixth event. In some examples, the sixth event can be a command for exiting the privacy mode inputted by a user to the mobile terminal. In some other examples, the sixth event can be reaching a preset end time for visit. These embodiments will be described in detail below.

In an embodiment, the mobile terminal controls at least one of the plurality of monitoring devices 100 to exit the privacy mode, in response to a sixth event, includes the mobile terminal controls at least one of the plurality of monitoring devices 100 (such as one or more monitoring devices 100) to exit the privacy mode, in response to a command for exiting the privacy mode inputted by a user to the mobile terminal. In one embodiment, the command for exiting the privacy mode is generated by triggering a physical or virtual key of the mobile terminal. Or the command for exiting the privacy mode is generated by recognizing the voice of the user by the mobile terminal. Or the command for entering into the privacy mode is generated by shaking the mobile terminal. Or the command for entering into the privacy mode is generated by recognizing the touching gesture of the user by the mobile terminal. For example, the central monitoring system provides a physical or virtual key. When the user presses the key, the mobile terminal controls at least one of the plurality of monitoring devices 100 to exit the privacy mode. For another example, the user sends a voice to the mobile terminal, such as "controlling the monitoring device to exit the privacy mode". After recognizing the voice, the mobile terminal controls at least one of the plurality of monitoring devices 100 to exit the privacy mode. For another example, after the user shakes the mobile terminal, the mobile terminal controls at least one of the plurality of monitoring devices 100 to exit the privacy mode. For a further example. After recognizing the preset touching gesture of the user, the mobile terminal controls at least one of the plurality of monitoring devices 100 to exit the privacy mode. It can be understood that in some schemes, when the monitoring device 100 is not in the privacy mode, the mobile terminal controls the monitoring device 100 to enter into the privacy mode after the user shakes the mobile terminal. When the monitoring device 100 is in the privacy mode, the mobile terminal controls the monitoring device 100 to exit the privacy mode after the user shakes the mobile terminal. That is, the user enables the monitoring device 100 to enter into the privacy mode by shaking the mobile terminal, and then enables the monitoring device 100 to exit the privacy mode by shaking the mobile terminal again.

In one embodiment, the mobile terminal controls at least one of the plurality of monitoring devices 100 to exit a privacy mode, in response to a sixth event, includes that the mobile terminal controls at least one of the plurality of monitoring devices 100 to exit a privacy mode, in response to reaching a preset end time for visit. For example, the preset visit end time is 7 p.m. When it is 7 p.m., the mobile terminal automatically controls at least one of the plurality of monitoring devices 100 to exit the privacy mode.

Referring Fig.11, one embodiment of this disclosure has disclosed a mobile terminal, such as a mobile phone or a tablet computer and so on, which includes: a communication interface 220, a memory 231 and a processor 232. The communication interface 220 is operable to communicationally connect with a plurality of monitoring devices 100. The memory 231 is operable to store a program. The processor 232 is operable to execute the program stored in the memory to implement the patient monitoring method for a mobile terminal, recited in any one embodiment discussed herein.

In some schemes, the monitoring device 100 can also automatically enter into or exit the privacy mode, as described in detail below.

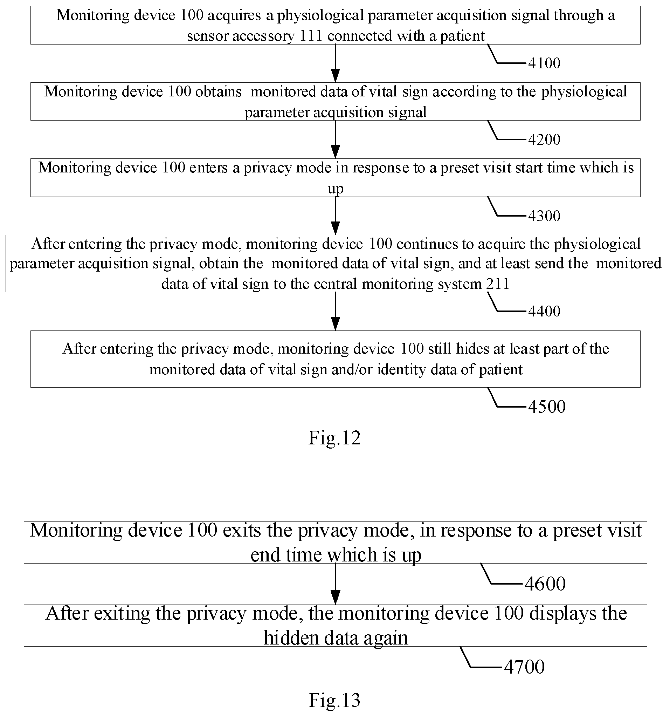

Referring Fig.12, one embodiment of this disclosure has disclosed a patient monitoring method for a medical monitoring device, which is in communication connection with a central monitoring system 211. The patient monitoring method for a medical monitoring device includes steps 4100 and 4500.

In step 4100, the monitoring device 100 acquires a physiological parameter acquisition signal through a sensor accessory 111 connected with a patient.

In step 4200, the monitoring device 100 obtains monitored data of vital sign according to the physiological parameter acquisition signal.

In step 4300, the monitoring device 100 enters into a privacy mode in response to reaching a preset start time for visit.

In step 4400, after entering into the privacy mode, the monitoring device 100 continues to use the sensor accessory 111 connected with the patient to acquire the physiological parameter acquisition signal, obtain the monitored data of vital sign according to the physiological parameter acquisition signal, and at least send the monitored data of vital sign to the central monitoring system 211.

In step 4500, after entering into the privacy mode, the monitoring device 100 still hides at least part of the monitored data of vital sign and/or identity data of patient. For example, the monitoring device 100 can hide the monitored data of vital sign and display the identity data of patient.

Referring Fig. 13, in one embodiment of this disclosure the patient monitoring method for a medical monitoring device further includes steps 4600 and 4700.

In step 4600, the monitoring device 100 exits the privacy mode, in response to reaching a preset end time for visit.

In step 4700, after exiting the privacy mode, the monitoring device 100 displays the hidden data again.

As can be seen from the above description, a variety of schemes for controlling the monitoring device 100 to enter into the privacy mode, for example, controlling one or more monitoring devices 100 to enter into the privacy mode through the central monitoring system 211; for example, controlling one or more neighbor bed monitoring devices to enter into the privacy mode through the monitoring device; for example, controlling one or more monitoring devices 100 to enter into the privacy mode through the mobile terminal; for example, enabling the monitoring device 100 to automatically enter into the privacy mode in response to the preset visitation start time which is up, are disclosed. Similarly, a variety of schemes for controlling the monitoring device 100 to exit the privacy mode, for example, controlling one or more monitoring devices 100 to exit the privacy mode through the central monitoring system 211; for example, controlling one or more neighbor bed monitoring devices to exit the privacy mode through the monitoring device; for example, controlling one or more monitoring devices 100 to exit the privacy mode through the mobile terminal; for example, enabling the monitoring device 100 to automatically exit the privacy mode in response to the preset visitation end time which is up, are disclosed. One skilled in the art can understand that one or more of the variety of schemes for controlling the monitoring device 100 to enter into the privacy mode can be freely combined with one or more of the variety of schemes for controlling the monitoring device 100 to exit the privacy mode. The schemes obtained by these free combinations are disclosed in the schemes recorded in this disclosure.

This disclosure also makes some improvements to the function of the privacy mode of the monitoring device 100, which will be described in detail below.

In one embodiment, the patient monitoring method disclosed in any embodiment of this disclosure may also include the following contents.

The monitoring device 100 may generate and display an area for the user to inputted a password in response to a command for entering into the privacy mode inputted by the user to the monitoring device 100. The monitoring device 100 determines whether the password is correct, in response to the password inputted by the user in the area. When the password is correct, the monitoring device 100 enters into the privacy mode. On the contrary, the monitoring device 100 does not enter into the privacy mode. This can prevent the monitoring device 100 from entering into the privacy mode caused by some misoperation or carelessness.

In order to ensure the safety of the monitoring device 100 after entering into the privacy mode, in one embodiment, the monitoring device 100 can determine whether it is in communication connection with the central monitoring system 211, in response to a command for entering into the privacy mode inputted by the user to the monitoring device 100. If the monitoring device 100 is in communication connection with the central monitoring system 211, the monitoring device 100 enters into the privacy mode, otherwise, the monitoring device 100 does not enter into the privacy mode. In such a way, the normal monitoring and alarm can be maintained. When some monitoring devices 100 are in the privacy mode, the monitored information of the patient cannot be displayed in real time on the monitoring device 100, and the audible and visual indications during the alarm is also hidden. At this time, the patient monitoring completely depends on the central station system. Therefore, if the monitoring device 100 enters into the privacy mode when it is disconnected from the central monitoring system 211, some data hidden and displayed by the monitoring device 100 will be in a state that cannot be viewed, which is very unsafe for the patient monitoring.

Similarly, in order to ensure the safety of patient monitoring, in one embodiment, after entering into the privacy mode, when the monitoring device 100 determines that its communication connection with the central monitoring system is disconnected, it exits the privacy mode, so that normal monitoring, alarm, etc. can be carried out.

In some embodiments, the central monitoring system 211 described above can be configured with one or more display screens, and the contents and data displayed or hidden by each display screen can be preset. Similarly, in an embodiment, the monitoring device 100 can also be configured with one or more display screens. For example, for some serious or isolated wards, it is very necessary and practical to configure one display screen in the room, and to configure one display screen at the door outside the room. The monitoring device 100 receives a command for patient privacy setting inputted by the user to the monitoring device 100. The command for patient privacy setting is used to enable the monitoring device 100 to select the corresponding information in the identity data of patient to display and/or hide. Therefore, in response to the command for patient privacy setting, the monitoring device 100 selects the corresponding information in the identity data of patient for one or more display screens to display and/or hide. As described above, the identity data of patient is some data used to represent the patient's own identity, such as one or more items of the information about the identity of patient, such as ID, name, age and bed number of the patient. Therefore, the medical staff can select which information in the identity data of patient to be displayed for each display screen of the monitoring device 100. For example, the medical staff can set the display screens configured in public areas such as aisles and corridors to display only the patient ID in the identity data of patient, or to hide and do not display the patient ID and patient name, while set the display screen configured in the patient room to display all the information in the identity data of patient and do not hide any information in the identity data of patient.

In one embodiment, the monitoring device 100 (such as, monitoring device A) can also enter into a neighbor bed observation mode, in response to a command for entering into the neighbor bed observation mode inputted by the user to the monitoring device 100 (such as, monitoring device A). During the neighbor bed observation mode, the monitoring device 100 displays the monitored data of vital sign and/or identity data of patient of a neighbor monitoring device 100 (such as, monitoring device B) corresponding to the neighbor bed observation mode. Wherein the identity data of patient includes at least name of patient. The monitoring device 100 (such as, monitoring device A) hides the name of patient in the neighbor bed observation mode (the name of patient who is monitored by the monitoring device B), in response to a command for hiding the name of patient inputted by the user.

One embodiment of this disclosure has disclosed a monitoring device 100, which includes a signal acquisition circuit 112, a processor, a memory 118 and a display 119. The processor is operable to obtain monitored data of vital sign according to the physiological parameter acquisition signal acquired by the signal acquisition circuit 112. The monitored data of vital sign at least includes physiological parameter values and physiological waveform data. In one embodiment, the processor can be implemented by the main processor 115, or in one embodiment, the processor can include the main processor 115 and the signal processing circuit 113. The memory 118 stores at least the obtained physiological parameter values and physiological waveform data. The display 119 outputs and displays at least physiological parameter values and physiological waveform data. In one embodiment, the memory 118 is also used to store a program, and the processor in the monitoring device 100 is also used to execute the program stored in the memory 118 to realize the patient monitoring method for the medical monitoring device and some improved functions related to the privacy mode of the monitoring device 100 according to any embodiment herein.

The description has been made with reference to various exemplary embodiments herein. However, those skilled in the art will recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communication connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A patient monitoring method for an in-hospital central monitoring system, **characterised in that**, the method comprises:
acquiring data of a plurality of monitoring devices by the central monitoring system, wherein the acquired data comprises monitored data of vital sign and identity data of patient;
displaying at least part of the acquired data of the plurality of monitoring devices by the central monitoring system;
controlling at least one of the plurality of monitoring devices to enter into a privacy mode by the central monitoring system, in response to a first event; and
continuing to acquire and display data of the plurality of monitoring devices by the central monitoring system.

2. The method according to claim 1, **characterised in that**, controlling at least one of the plurality of monitoring devices to enter into a privacy mode by the central monitoring system, in response to a first event, comprises:
controlling at least one of the plurality of monitoring devices to enter into the privacy mode by the central monitoring system, in response to a command for entering into the privacy mode inputted by a user to the central monitoring system.

3. The method according to claim 2, **characterised in that**, the command for entering into the privacy mode is generated by triggering a physical or virtual key of the central monitoring system, or the command for entering into the privacy mode is generated by recognizing gesture or voice of the user by the central monitoring system.

4. The method according to claim 1, **characterised in that**, controlling at least one of the plurality of monitoring devices to enter into a privacy mode by the central monitoring system, in response to a first event, comprises:
controlling at least one of the plurality of monitoring devices to enter into the privacy mode by the central monitoring system, in response to reaching a preset start time for visit.

5. The method according to claim 1, **characterised in that**, the central monitoring system is configured with one or more display screens, and the identity data of patient includes one or more of information for bed number of patient, information for name of patient, and information for ID of patient; wherein the method further comprises:
receiving, by the central monitoring system, a command for patient privacy setting inputted by a user; wherein the command for patient privacy setting enables the central monitoring system to select corresponding information in the identity data of patient for displaying and/or hiding; and
selecting the corresponding information in the identity data of patient for the one or more display screens to display and/or hide by the central monitoring system, in response to the command for patient privacy setting.

6. The method according to claim 5, **characterised in that**, the central monitoring system provides a physical or virtual key; wherein, when the information for name of patient is selected to be hidden, the method further comprises:
displaying the information for name of patient by the central monitoring system, when the central monitoring system determines that the key is pressed; and continuing to hide the information for the name of patient by the central monitoring system, when the central monitoring system determines that the key is not pressed; or
displaying the information for name of patient by the central monitoring system, when the central monitoring system receives a signal that the key is triggered, during a time when the central monitoring system hides the information for name of patient; and hiding the information for name of patient by the central monitoring system, when the central monitoring system receives a signal that the key is triggered, during a time when the central monitoring system displays the information for name of patient.

7. The method according to claim 6, **characterised in that**, the method further comprises:
enabling the key by the central monitoring system, in response to an enabling command; and
disabling the key by the central monitoring system, in response to a disabling command.

8. The method according to any one of claims 1-7, **characterised in that**, the method further comprises:
controlling at least one of the plurality of monitoring devices to exit the privacy mode by the central monitoring system, in response to a second event.

9. The method according to claim 8, **characterised in that**, controlling at least one of the plurality of monitoring devices to exit the privacy mode by the central monitoring system, in response to a second event, comprises:
controlling at least one of the plurality of monitoring devices to exit the privacy mode by the central monitoring system, in response to a command for exiting the privacy mode inputted by a user to the central monitoring system.

10. The method according to claim 9, **characterised in that**, the command for exiting the privacy mode is generated by triggering a physical or virtual key of the central monitoring system, or the command for exiting the privacy mode is generated by recognizing gesture or voice of the user by the central monitoring system.

11. The method according to claim 8, **characterised in that**, controlling at least one of the plurality of monitoring devices to exit the privacy mode by the central monitoring system, in response to a second event, comprises:
controlling at least one of the plurality of monitoring devices to exit the privacy mode by the central monitoring system, in response to reaching a preset end time for visit.

12. A patient monitoring method for a medical monitoring device which is connected with a central monitoring system, **characterised in that**, the method comprises:
acquiring a physiological parameter acquisition signal through a sensor accessory connected with a patient, by the monitoring device;
obtaining monitored data of vital sign according to the physiological parameter acquisition signal, by the monitoring device;
enabling the monitoring device to enter into a privacy mode in response to a first command that is sent by the central monitoring system in response to a first event, wherein the central monitoring system responds to the first event to control one or more monitoring devices to enter into the privacy mode;
continuing to use the sensor accessory connected with the patient to acquire a physiological parameter acquisition signal, obtain monitored data of vital sign according to the physiological parameter acquisition signal, and at least send the monitored data of vital sign to the central monitoring system, by the monitoring device, after entering into the privacy mode; and
hiding at least part of the monitored data of vital sign and/or identity data of patient, by the monitoring device, after entering into the privacy mode.

13. The method according to claim 12, **characterised in that**, the method further comprises:
generating and displaying an area for a user to input a password by the monitoring device, in response to a command for entering into the privacy mode inputted by the user to the monitoring device; and
determining whether the password is correct by the monitoring device, in response to the password inputted by the user in the area; and enabling the monitoring device to enter into the privacy mode when the password is correct and not to enter into the privacy mode when the password is incorrect.

14. The method according to claim 12, **characterised in that**, the method further comprises:
determining whether the monitoring device is in communication connection with the central monitoring system by the monitoring device, in response to a command for entering into the privacy mode inputted by the user to the monitoring device; and
enabling the monitoring device to enter into the privacy mode when the monitoring device is in communication connection with the central monitoring system and not to enter into the privacy mode when the monitoring device is not in communication connection with the central monitoring system.

15. The method according to any one of claims 12-14, **characterised in that**, the method further comprises:
enabling the monitoring device to exit the privacy mode when the monitoring device determines that a communication connection between the monitoring device and the central monitoring system is disconnected, after entering into the privacy mode.

16. The method according to claim 12, **characterised in that**, the monitoring device is configured with one or more display screens, and the identity data of patient includes one or more of information for bed number of patient, information for name of patient, and information for ID of patient; wherein the method further comprises:
receiving, by the monitoring device, a command for patient privacy setting inputted by a user, wherein the command for patient privacy setting enables the monitoring device to select corresponding information in the identity data of patient for displaying and/or hiding; and
selecting the corresponding information in the identity data of patient for the one or more display screens to display and/or hide by the monitoring device, in response to the command for patient privacy setting.

17. The method according to claim 12, **characterised in that**, the method further comprises:
enabling the monitoring device to enter into a neighbor bed observation mode in response to a command for entering into the neighbor bed observation mode inputted by a user to the monitoring device;
displaying monitored data of vital sign and/or identity data of patient from a neighbor monitoring device corresponding to the neighbor bed observation mode by the monitoring device, when entering into the neighbor bed observation mode, wherein the identity data of patient comprises at least name of patient; and
hiding the name of patient in the neighbor bed observation mode by the monitoring device, in response to a command for hiding the name of patient inputted by the user.

18. The method according to claim 12, **characterised in that**, hiding at least part of the monitored data of vital sign and/or identity data of patient, by the monitoring device, after entering into the privacy mode, comprises:
hiding the monitored data of vital sign and displaying the identity data of patient, by the monitoring device, after entering into the privacy mode.

19. The method according to any one of claims 12-18, **characterised in that**, the method further comprises:
enabling the monitoring device to exit the privacy mode in response to a second command that is sent by the central monitoring system in response to a second event, wherein the central monitoring system responds to the second event to control at least one of the monitoring devices to exit the privacy mode; and
displaying the hidden data again by the monitoring device after exiting the privacy mode.

20. The method according to any one of claims 12-18, **characterised in that**, the method further comprises:
enabling the monitoring device to exit the privacy mode, in response to reaching a preset end time for visit; and
displaying the hidden data again by the monitoring device after exiting the privacy mode.

21. A patient monitoring method for a medical monitoring device which is in communication connection with a central monitoring system, **characterised in that**, the method comprises:
acquiring a physiological parameter acquisition signal through a sensor accessory connected with a patient, by the monitoring device;
obtaining monitored data of vital sign according to the physiological parameter acquisition signal, by the monitoring device;
enabling the monitoring device to enter into a privacy mode in response to reaching a preset start time for visit;
continuing to use the sensor accessory connected with the patient to acquire the physiological parameter acquisition signal, obtain the monitored data of vital sign according to the physiological parameter acquisition signal, and at least send the monitored data of vital sign to the central monitoring system, by the monitoring device, after entering into the privacy mode; and
hiding at least part of the monitored data of vital sign and/or identity data of patient, by the monitoring device, after entering into the privacy mode.

22. The method according to claim 20, **characterised in that**, the method further comprises:
generating and displaying an area for a user to input a password by the monitoring device, in response to a command for entering into the privacy mode inputted by the user; and
determining whether the password is correct by the monitoring device, in response to the password inputted by the user in the area; and enabling the monitoring device to enter into the privacy mode when the password is correct and not to enter into the privacy mode when the password is incorrect.

23. The method according to claim 20, **characterised in that**, the method further comprises:
determining whether the monitoring device is in communication connection with the central monitoring system by the monitoring device, in response to a command for entering into the privacy mode inputted by the user to the monitoring device; and
enabling the monitoring device to enter into the privacy mode when the monitoring device is in communication connection with the central monitoring system and not to enter into the privacy mode when the monitoring device is not in communication connection with the central monitoring system.

24. The method according to any one of claims 20-22, **characterised in that**, the method further comprises:
enabling the monitoring device to exit the privacy mode when the monitoring device determines that a communication connection between the monitoring device and the central monitoring system is disconnected, after entering into the privacy mode.

25. The method according to claim 20, **characterised in that**, the monitoring device is configured with one or more display screens, and the identity data of patient includes one or more of information for bed number of patient, information for name of patient, and information for ID of patient; wherein the method further comprises:
receiving, by the monitoring device, a command for patient privacy setting inputted by a user, wherein the command for patient privacy setting enables the monitoring device to select corresponding information in the identity data of patient for displaying and/or hiding; and
selecting the corresponding information in the identity data of patient for the one or more display screens to display and/or hide by the monitoring device, in response to the command for patient privacy setting.

26. The method according to claim 20, **characterised in that**, the method further comprises:
enabling the monitoring device to enter into a neighbor bed observation mode, in response to a command for entering into the neighbor bed observation mode inputted by the user to the monitoring device;
displaying the monitored data of vital sign and/or identity data of patient of a neighbor monitoring device corresponding to the neighbor bed observation mode by the monitoring device, when entering into the neighbor bed observation mode, wherein the identity data of patient comprises at least name of patient; and
hiding the name of patient in the neighbor bed observation mode by the monitoring device, in response to a command for hiding the name of patient inputted by the user.

27. The method according to claim 21, **characterised in that**, hiding at least part of the monitored data of vital sign and/or identity data of patient, by the monitoring device, after entering into the privacy mode, further comprises:
hiding the monitored data of vital sign and displaying the identity data of patient, by the monitoring device, after entering into the privacy mode.

28. The method according to any one of claims 21-27, **characterised in that**, the method further comprises:
enabling the monitoring device to exit the privacy mode in response to a second command that is sent by the central monitoring system in response to a second event, wherein the central monitoring system responds to the second event to control at least one of the monitoring devices to exit the privacy mode; and
displaying the hidden data again by the monitoring device after exiting the privacy mode.

29. The method according to any one of claims 21-27, **characterised in that**, the method further comprises:
enabling the monitoring device to exit the privacy mode, in response to reaching a preset end time for visit; and
displaying the hidden data again by the monitoring device after exiting the privacy mode.

30. A patient monitoring method for a medical monitoring device which is in communication connection with a central monitoring system, **characterised in that**, the method comprises:
acquiring data of one or more neighbor bed monitoring devices by the monitoring device, wherein the data comprises monitored data of vital sign and identity data of patient;
displaying at least part of the acquired data of the neighbor bed monitoring devices, by the monitoring device;
controlling at least one of the neighbor bed monitoring devices to enter into a privacy mode by the monitoring device, in response to a third event; and
continuing to acquire and display the data of the neighbor bed monitoring devices by the monitoring device.

31. The method according to claim 30, **characterised in that**, controlling at least one of the neighbor bed monitoring devices to enter into a privacy mode by the monitoring device, in response to a third event, comprises:
controlling at least one of the neighbor bed monitoring devices to enter into the privacy mode by the monitoring device, in response to a command for entering into the privacy mode inputted by a user to the monitoring device.

32. The method according to claim 31, **characterised in that**, the command for entering into the privacy mode is generated by triggering a physical or virtual key of the monitoring device, or the command for entering into the privacy mode is generated by recognizing gesture or voice of the user by the monitoring device.

33. The method according to claim 30, **characterised in that**, controlling at least one of the neighbor bed monitoring devices to enter into a privacy mode by the monitoring device, in response to a third event, comprises:
controlling at least one of the neighbor bed monitoring devices to enter into the privacy mode by the monitoring device, in response to reaching a preset start time for visit.

34. The method according to any one of claims 30-33, **characterised in that**, the method further comprises:
controlling at least one of the neighbor bed monitoring devices to exit the privacy mode by the monitoring device, in response to a fourth event.

35. The method according to claim 34, **characterised in that**, controlling at least one of the neighbor bed monitoring devices to exit the privacy mode by the monitoring device, in response to a fourth event, comprises:
controlling at least one of the neighbor bed monitoring devices to exit the privacy mode by the monitoring device, in response to a command for exiting the privacy mode inputted by a user to the monitoring device.

36. The method according to claim 35, **characterised in that**, the command for exiting the privacy mode is generated by triggering a physical or virtual key of the monitoring device, or the command for exiting the privacy mode is generated by recognizing gesture or voice of the user by the monitoring device.

37. The method according to claim 34, **characterised in that**, controlling at least one of the neighbor bed monitoring devices to exit the privacy mode by the monitoring device, in response to a fourth event, comprises:
controlling at least one of the neighbor bed monitoring devices to exit the privacy mode by the monitoring device, in response to reaching a preset end time for visit.

38. A patient monitoring method for a mobile terminal which is in communication connection with one or more monitoring devices, **characterised in that**, the method comprises:
controlling at least one of the monitoring devices to enter into a privacy mode by the mobile terminal, in response to a fifth event;
wherein after entering into the privacy mode, the monitoring devices continue to send data to a central monitoring system, such that the data still can be displayed in the central monitoring system.

39. The method according to claim 38, **characterised in that**, controlling at least one of the monitoring devices to enter into a privacy mode by the mobile terminal, in response to a fifth event, comprises:
controlling at least one of the monitoring devices to enter into the privacy mode by the mobile terminal, in response to a command for entering into the privacy mode inputted by a user to the mobile terminal.

40. The method according to claim 39, **characterised in that**, the command for entering into the privacy mode is generated by triggering a physical or virtual key of the mobile terminal, the command for entering into the privacy mode is generated by recognizing voice of the user by the mobile terminal, the command for entering into the privacy mode is generated by shaking the mobile terminal, or the command for entering into the privacy mode is generated by recognizing touching gesture of the user by the mobile terminal.

41. The method according to claim 38, **characterised in that**, controlling at least one of the monitoring devices to enter into a privacy mode by the mobile terminal, in response to a fifth event, comprises:
controlling at least one of the monitoring devices to enter into the privacy mode by the mobile terminal, in response to reaching a preset start time for visit.

42. The method according to any one of claims 38-41, **characterised in that**,the method further comprises:
controlling at least one of the monitoring devices to exit the privacy mode by the mobile terminal, in response to a sixth event.

43. The method according to claim 42, **characterised in that**, controlling at least one of the monitoring devices to exit the privacy mode by the mobile terminal, in response to a sixth event, comprises:
controlling at least one of the monitoring devices to exit the privacy mode by the mobile terminal, in response to a command for exiting the privacy mode inputted by a user to the mobile terminal.

44. The method according to claim 43, **characterised in that**, the command for exiting the privacy mode is generated by triggering a physical or virtual key of the mobile terminal, the command for exiting the privacy mode is generated by recognizing voice of the user by the mobile terminal, the command for exiting the privacy mode is generated by shaking the mobile terminal, or the command for exiting the privacy mode is generated by recognizing touching gesture of the user by the mobile terminal.

45. The method according to claim 42, **characterised in that**, controlling at least one of the monitoring devices to exit the privacy mode by the mobile terminal, in response to a sixth event, comprises:
controlling at least one of the monitoring devices to exit the privacy mode by the mobile terminal, in response to reaching a preset end time for visit.

46. An in-hospital central monitoring system, **characterised in that**, comprising:
a communication interface which is operable to establish a communication connection with a plurality of monitoring devices;
a memory which is operable to store data of the plurality of monitoring devices acquired through the communication interface and to store a program, wherein the data comprises monitored data of vital sign and identity data of patient;
a display which is operable to display the acquired data of the monitoring devices; and
a processor which is operable to execute the program stored in the memory to implement the method according to any one of claims 1-11.

47. A medical monitoring device, **characterised in that**, comprising:
a signal acquisition circuit which is operable to use a sensor accessory connected with a patient to acquire a physiological parameter acquisition signal;
a processor which is operable to obtain monitored data of vital sign according to the physiological parameter acquisition signal acquired by the signal acquisition circuit;
a memory which is operable to store identity data of patient and the monitored data of vital sign; and
a display which is operable to display data;
wherein the memory is further operable to store a program and the processor is further operable to execute the program stored in the memory to implement the method according to any one of claims 12-37.

48. A mobile terminal, **characterised in that**, comprising:
a communication interface which is operable to communicationally connect with one or more monitoring devices;
a memory which is operable to store a program; and
a processor which is operable to execute the program stored in the memory to implement the method according to any one of claims 38-45.

49. A computer readable storage medium, **characterised in that**, comprising a program that can be executed by a processor to implement the method according to any one of claims 1-45.
